# EUROPÄISCHE PATENTSCHRIFT

(11) **EP 3 208 477 B1**
(45) Veröffentlichungstag und Bekanntmachung des Hinweises auf die Patenterteilung: **04.08.2021**
(21) Anmeldenummer: 16156328.3
(22) Anmeldetag: 18.02.2016
(51) Int. Cl.: F16B 35/06, F16B 39/284, F16B 39/32

(54) **SATZ VON BEFESTIGUNGSELEMENT ZUM VERHINDERN VON UNGEWOLLTEM LÖSEN EINER VERBINDUNG UND GEGENSTÜCK SOWIE VERWENDUNG DES SATZES**
SET OF FASTENING ELEMENT FOR PREVENTING UNWANTED RELEASE OF A CONNECTION AND COUNTERPART AND USE OF THE SET
ENSEMBLE D'ELEMENT DE FIXATION DESTINE A EMPECHER LE DETACHEMENT INVOLONTAIRE D'UNE LIAISON ET PENDANT ET UTILISATION DE L'ENSEMBLE

(43) Veröffentlichungstag der Anmeldung: 23.08.2017
(73) Patentinhaber: WALDEMAR LINK GmbH & Co. KG, 22339 Hamburg (DE)
(72) Erfinder: DMUSCHEWSKY, Klaus, 22397 Hamburg (DE)
(74) Vertreter: Hoffmann Eitle

(56) Entgegenhaltungen:
- WO-A1-2009/001421
- WO-A1-2009/012195
- WO-A2-00/73668
- US-A1- 2011 082 506
- US-A1- 2015 216 573
- None

## Beschreibung

Die Erfindung betrifft ein Befestigungselement zum Verhindern von ungewolltem Lösen einer Verbindung zwischen dem Befestigungselement und einem Gegenstück. Das Befestigungselement ist so eingerichtet, dass es in Eingriff mit dem Gegenstück über eine Drehbewegung des Befestigungselements um eine Drehachse in einer Verbindungsrichtung kommen kann. Derartige Befestigungselemente sind geeignet zur Anwendung in den verschiedensten technischen Bereichen, bspw. der Automobilindustrie, der Haushaltselektronik und der Medizintechnik, insbesondere im Bereich von Instrumenten für Implantate bzw. Prothesen und von modularen Systemen von Implantaten bzw. Prothesen.

### Stand der Technik

Befestigungselemente sind bekannt. Beispielsweise offenbaren die US 2011/0082506 A1 und die US 2015/0216573 A1 eine Befestigungsanordnung zum Stabilisieren von Wirbelkörpern mit einer Knochenschraube, die ein Gewinde und einen Kopf aufweist, wobei sich flexible Beine von dem Zentrum wegerstrecken, die dazu dienen, eine versehentliche Bewegung des Schraubenkopfes zu verhindern. Ebenfalls ein System zum Stabilisieren von Wirbelsäulen offenbart die WO 2009/012195 A1.

Bei Befestigungselementen werden jedoch immer höhere Anforderungen dahingehend gestellt, dass sich das Befestigungselement nicht ungewollt lösen soll, nachdem die Verbindung zwischen dem Befestigungselement und dem Gegenstück hergestellt worden ist. Insbesondere sind keine verlässlichen Befestigungselemente bekannt, die auch bei Vibrationen oder Wechsellasten zuverlässig ein ungewolltes Lösen verhindern.

Beispielsweise offenbaren die WO 2009/001421 A1 und die WO 00/73668 A2 Schraubenanordnungen, die ein Lösen einer Schraubenverbindung durch ineinandergreifende Elemente verhindern sollen.

### Darstellung der Erfindung

Es ist Aufgabe der vorliegenden Erfindung, bei einfacher Konstruktion ein Befestigungselement bzw. einen Satz aus einem Befestigungselement und einem Gegenstück bzw. eine Verwendung eines Befestigungselements derart zu gestalten, dass ein ungewolltes Lösen der Verbindung zwischen dem Befestigungselement und dem Gegenstück auf verlässliche Weise verhindert werden kann.

Diese Aufgabe wird erfindungsgemäß durch den Satz aus einem Befestigungselement und einem Gegenstück mit den Merkmalen von Anspruch 1 gelöst. Insbesondere ist ein Befestigungselement zum Verhindern von ungewolltem Lösen einer Verbindung zwischen dem Befestigungselement und einem Gegenstück vorgesehen, wobei das Befestigungselement eingerichtet ist, in Eingriff mit dem Gegenstück über eine Drehbewegung des Befestigungselements um eine Drehachse in einer Verbindungsrichtung zu kommen. Das Befestigungselement weist ein elastisches Element, insbesondere ein Federelement, mit einem Kontaktabschnitt zum Kontaktieren des Gegenstücks auf, wobei das elastische Element derart ausgebildet ist, dass es zumindest abschnittsweise, in einem vorgespannten und/oder nicht-vorgespannten Zustand, winkelig zu einer auf die Drehachse bezogenen Radialen in einer Ebene senkrecht zur Drehachse angeordnet ist.

Erfindungsgemäß ist ein Satz aus dem erfindungsgemäßen Befestigungselement und dem Gegenstück vorgesehen, wobei das Befestigungselement und das Gegenstück derart ausgebildet sind, dass zumindest im verbundenen Zustand des Befestigungselements und des Gegenstücks der Kontaktabschnitt das Gegenstück in einem Kontaktpunkt kontaktiert und dass eine auf die Drehachse bezogene Tangente im Kontaktpunkt zumindest zu einem Abschnitt des elastischen Elements in einer Ebene senkrecht zu der Drehachse in einem Winkel ungleich 90° verläuft.

Gemäß Anspruch 14 ist die Verwendung eines Befestigungselements zum Verhindern von ungewolltem Lösen einer Verbindung zwischen dem Befestigungselement und einem Gegenstück vorgesehen, wobei das Befestigungselement insbesondere eingerichtet ist, in Eingriff mit dem Gegenstück über eine Drehbewegung des Befestigungselements um eine Drehachse in einer Verbindungsrichtung zu kommen, wobei das Befestigungselement ein elastisches Element, insbesondere Federelement, mit einem Kontaktabschnitt zum Kontaktieren des Gegenstücks in einem Kontaktpunkt aufweist, und der Kontaktabschnitt das Gegenstück im verbundenen Zustand des Befestigungselements und des Gegenstücks kontaktiert, und das elastische Element im verbundenen Zustand derart angeordnet ist, dass eine auf die Drehachse bezogene Tangente im Kontaktpunkt zumindest zu einem Abschnitt des elastischen Elements in einer Ebene senkrecht zu der Drehachse in einem Winkel ungleich 90° verläuft.

Gemäß Anspruch 15 ist die Verwendung des erfindungsgemäßen Satzes zur Verbindung von Implantatkomponenten, insbesondere einer (winkelstabilen) Knochenplatte, vorgesehen.

Unter der Radialen ist eine Gerade zu verstehen, die sich in einer Ebene senkrecht zur Drehachse radial von der Drehachse wegersteckt.

Als verbundener Zustand ist insbesondere der Zustand zu verstehen, in dem das Befestigungselement mit dem Gegenstück derart in Eingriff ist, dass der Eingriffsabschnitt des Befestigungselements und der Eingriffsabschnitt des Gegenstücks vollständig ineinander aufgenommen sind und dabei die kraftschlüssige Verbindung erreicht ist.

Das elastische Element kann integral mit dem Befestigungselement ausgebildet sein oder kann separat an dem Befestigungselement angeordnet sein. Der Bereich des Gegenstücks, der mit dem Befestigungselement in Eingriff kommt, kann integral oder separat zu dem Bereich des Gegenstücks sein, mit dem der Kontaktabschnitt des Befestigungselements in Kontakt kommt. Es können beispielsweise ein erstes und ein zweites Gegenstück vorgesehen sein, wobei das erste Gegenstück zum Eingriff mit dem Befestigungselement durch die Drehbewegung dient (indem es beispielsweise ein Gewinde aufweist) und das zweite Gegenstück zum Kontakt mit dem elastischen Element dient. Es ist denkbar, dass zwischen dem ersten und zweiten Gegenstück ein zu befestigendes Element vorgesehen ist, das durch die Verbindung zwischen dem Befestigungselement und dem Gegenstück an dem Gegenstück befestigt wird. Es ist auch möglich, dass das Befestigungselement mit dem Gegenstück verbunden wird und dabei ein weiteres Objekt arretiert, indem das Befestigungselement durch das Gegenstück hindurch in das weitere Objekt eingreift, und so verhindert, dass das Objekt aus dem Gegenstück entfernt werden kann.

Das Gegenstück kann aus zwei getrennten Abschnitten bestehen. Ein Abschnitt des Gegenstücks kann mit dem anderen Abschnitt des Gegenstücks mit Hilfe des Befestigungselements verbunden bzw. befestigt werden. Mit anderen Worten kann das Gegenstück auch teilweise in dem zu befestigenden Objekt ausgebildet sein oder zusätzlich zu dem zu befestigenden Objekt vorgesehen sein.

Dadurch, dass das elastische Element winklig zur Radialen und bzw. in einem Winkel ungleich 90° relativ zu einer Tangente im Kontaktpunkt angeordnet ist, sind die radialen Kräfte beim Drehen des Befestigungselements in die Verbindungsrichtung und in eine zur Verbindungsrichtung entgegengesetzten Richtung unterschiedlich.

Der Erfindung liegt der Gedanke zugrunde, das ungewollte Lösen eines Befestigungselements von einem Gegenstück dadurch zu verhindern, dass zumindest das anfängliche Lösen der Verbindung zwischen dem Befestigungselement und dem Gegenstück einen stark erhöhten Kraftaufwand erfordert, der höher als die zusätzliche Kraft ist, die beim Erzeugen der Verbindung zwischen dem Befestigungselement und dem Gegenstück auf Grund des elastischen Elements aufzubringen ist. Indem die notwendige Kraft beim zumindest anfänglichen Lösen erhöht ist, kann verhindert werden, dass es zu einem ungewollten Lösen der Verbindung zwischen dem Befestigungselement und dem Gegenstück kommt. Dies erhöht die Verlässlichkeit der Verbindung. Durch diese erhöhte zusätzliche notwendige Kraft, um die Verbindung zwischen dem Befestigungselement und dem Gegenstück anfänglich lösen zu können, kann das Befestigungselement auch im Falle von Vibrationen oder Wechsellasten gegen ein Lösen aus der Verbindung mit dem Gegenstück gesichert sein. Somit ist ein Objekt, das durch die Verbindung zwischen dem Befestigungselement und dem Gegenstück mit dem Gegenstück verbunden bzw. an ihm befestigt ist, gegen ein Lösen aus der Verbindung mit dem Gegenstück gesichert.

Mit anderen Worten kann das Befestigungselement beim Erzeugen der Verbindung zwischen dem Befestigungselement und dem Gegenstück im Wesentlichen ohne zusätzliche Kraft mit dem Gegenstück in Eingriff gebracht werden, wohingegen zumindest beim anfänglichen Drehen des Befestigungselements in die der Verbindungsrichtung entgegengesetzte Richtung eine derart hohe Kraft notwendig ist, dass dies im Wesentlichen einer Sperrung des Befestigungselements gleichkommt.

Besonders vorteilhafte Weiterbildungen der Erfindung sind in den abhängigen Ansprüchen und im Folgenden angegeben. Bevorzugt weist das Gegenstück eine Implantantkomponente auf. Es ist denkbar, dass ein Teil des Gegenstücks (für den Eingriff mit dem Befestigungselement) einem Knochen entspricht, an dem eine Knochenplatte zu befestigen ist. Die Knochenplatte kann einen weiteren Teil des Gegenstücks (für den Kontakt mit dem elastischen Element) aufweisen. Es ist auch denkbar, dass der weitere Teil des Gegenstücks separat zur Knochenplatte vorgesehen ist.

Insbesondere ist das elastische Element derart ausgebildet, dass durch den Kontakt des elastischen Elements mit dem Gegenstück die Kraft, die für den Eingriff mit dem Gegenstück bei Erzeugen der Verbindung nötig ist, nur leicht bzw. im Wesentlichen kaum erhöht wird, und derart, dass die Kraft, die zwischen dem Befestigungselement und dem Gegenstück zum Lösen der Verbindung nötig ist, durch den Kontakt des elastischen Elements mit dem Gegenstück stark erhöht ist.

Vorzugsweise ist das elastische Element derart ausgebildet, dass, nachdem die erhöhte Kraft beim anfänglichen Lösen der Verbindung aufgebracht worden ist, das Befestigungselement von dem Gegenstück gelöst werden kann, wobei nur eine geringe bzw. im Wesentlichen keine zusätzliche Kraft mehr nötig ist.

Vorzugsweise ist das elastische Element ein Federelement und derart ausgebildet, dass Vorspannung auf das elastische Element derart aufbringbar ist, dass Kontakt zwischen dem Kontaktabschnitt und dem Gegenstück in einem Kontaktpunkt sichergestellt werden kann. Durch die Vorspannung des elastischen Elements kann sichergestellt werden, dass die Rückstellkräfte das elastische Element gegen das Gegenstück ausreichend drücken, sodass der Kontakt zwischen dem Kontaktabschnitt und dem Gegenstück im Kontaktpunkt gewährleistet werden kann.

Weitervorzugsweise ist das elastische Element, vorzugsweise auch in einem nicht-vorgespannten Zustand, derart winklig zu einer Radialen in einer Ebene senkrecht zur Drehachse angeordnet, dass zumindest im Kontaktpunkt der Winkel zwischen dem elastischen Element und der Radialen in Verbindungsrichtung größer als 0° und kleiner als 90° beträgt. Mit anderen Worten ist, wenn die Verbindungsrichtung im Uhrzeigersinn verläuft, der Winkel zwischen dem elastischen Element im Kontaktpunkt hin zu der Radialen im Uhrzeigersinn größer als 0° und kleiner als 90°. Der Winkel zwischen dem elastischen Element und der Radialen in der der Verbindungsrichtung entgegengesetzten Richtung, also der Richtung entgegen dem Uhrzeigersinn, beträgt hingegen mehr als 270°. Dadurch kann erreicht werden, dass die aufgrund des elastischen Elements zusätzlich aufzubringende Kraft beim Drehen des Befestigungselements in Verbindungsrichtung geringer als die zusätzliche Kraft beim Drehen des Befestigungselements in die der Verbindungsrichtung entgegengesetzte Richtung ist.

Vorzugsweise beträgt der Winkel zwischen dem elastischen Element und der Radialen in Verbindungsrichtung zwischen 5° und 90°, weiter vorzugsweise zwischen 5° und 85°, weiter bevorzugt zwischen 5° und 45°.

Material und Oberfläche des elastischen Elements und des Gegenstückes werden bevorzugt so gewählt, dass das elastische Element zumindest im Kontaktbereich mit dem Gegenstück eine geeignete Elastizität und/oder Oberflächenrauigkeit aufweist, um eine kraftschlüssige Verbindung mit dem Gegenstück zu unterstützen. Das Material darf eine Gleitreibung nicht begünstigen und hat insbesondere elastische, federnde Eigenschaften. Bevorzugte Oberflächenrauigkeiten des elastischen Elements und des Gegenstückes in ihrem Kontaktbereich sind Rₐ = 0,005 - 25 pm. Bevorzugt ist das Material des elastischen Elements und/oder des Gegenstückes Kunststoff oder Metall. Weitere bevorzugte Beispiele sind Stähle (gehärtet oder ungehärtet), Titanlegierungen, Kobalt-Chrom, Messing, Bronze, Aluminiumlegierungen und verschiedene Kunststoffe (die federnde Eigenschaften aufweisen) wie PEEK (Polyetheretherketon; beispielsweise erhältlich als Zeniva® der Firma Solvay), Radel® (ein Polyphenylensulfon-Kunststoff der Firma Solvay) und Propylux® (ein Polypropylen-Kunststoff der Firma Westlake Plastics Company). Auch die Verwendung von Holz oder Gummi ist denkbar. Grundsätzlich ist eine Vielzahl von Kombinationen oder Werkstoffpaarungen möglich, in Abhängigkeit vom Anwendungsgebiet.

Vorzugsweise liegt der Winkel in Verbindungsrichtung zwischen der auf die Drehachse bezogene Tangente im Kontaktpunkt und zumindest einem Abschnitt des elastischen Elements in einer Ebene senkrecht zur Drehachse zwischen 5° und 85°, vorzugsweise zwischen 45° und 85.

Das elastische Element ist derart ausgebildet, dass eine Krafteinleitung in das elastische Element bei Drehung des Befestigungselements um die Drehachse in Verbindungsrichtung kleiner als bei Drehung des Befestigungselements in die der Verbindungsrichtung entgegengesetzte Richtung ist. Dabei wird erreicht, dass das elastische Element bei Drehung des Befestigungselements in die Verbindungsrichtung weniger zusätzlichen Kraftaufwand erfordert, da beispielsweise nur eine geringe Gleitreibungskraft zu überwinden ist, da sich das elastische Element entsprechend nachgiebig verformt. Beim Drehen des Befestigungselements hingegen in die der Verbindungsrichtung entgegengesetzte Richtung wird durch die Drehung des Befestigungselements in die der Verbindungsrichtung entgegengesetzte Richtung Kraft in das elastische Element derart eingeleitet, dass es im Wesentlichen versteift, und somit weniger elastisch ausgeprägt ist, sodass die im Kontaktpunkt zwischen dem Gegenstück und dem Kontaktabschnitt auftretende Reibungskraft wesentlich höher als die bei Drehung in Verbindungsrichtung aufzubringende Gleitreibungskraft ist, bevorzugt so hoch, dass Haftreibung auftritt.

Vorzugsweise ist die Richtung, in die das elastische Element vorspannbar ist, unterschiedlich zu einer Richtung, in die die Krafteinleitung in das elastische Element bei Drehung des Befestigungselements erfolgen kann. Weiter vorzugsweise ist die Richtung, in die das Element vorspannbar ist, senkrecht zu der Richtung, in die die Krafteinleitung in das elastische Element erfolgen kann; dadurch kann die Krafteinleitung und damit die Versteifung des elastischen Elements maximiert werden, was die zusätzliche Kraft beim anfänglichen Lösen der Verbindung stark erhöht und somit ein ungewolltes Lösen zuverlässiger verhindert.

Das elastische Element ist derart ausgebildet, dass der Kontaktabschnitt des vorgespannten elastischen Elements und das Gegenstück zumindest bei anfänglicher Drehung des Befestigungselements in die der Verbindungsrichtung entgegengesetzte Richtung kraftschlüssig verbunden sind. Mit anderen Worten soll zwischen dem Kontaktabschnitt und dem Gegenstück ein Reib- bzw. Kraftschluss vorliegen, der durch die radiale Kraft zustande kommt, die zwischen dem Kontaktabschnitt und dem Gegenstück wirkt. Dies ist im Gegensatz zu einem Formschluss zu sehen.

Vorzugsweise ist das elastische Element im Wesentlichen länglich, insbesondere als Bein, weiter insbesondere als Biegefeder, ausgebildet. Bei dieser Ausbildung ist die Krafteinleitung und damit das ungewollte Lösen besonders gut umzusetzen.

Das elastische Element kann Metall aufweisen.

Es kann eine Länge von mindestens 3 mm, vorzugsweise von mindestens 10 mm und/oder weniger als 20 mm aufweisen.

Das Befestigungselement kann einen Eingriffsabschnitt zum Eingriff mit dem Gegenstück aufweisen, wobei der Eingriffsabschnitt an einem Ende einen vorzugsweise zumindest abschnittsweise kreisförmigen Endabschnitt aufweist, an dem das elastische Element angeordnet ist. Wenn das elastische Element an einem Endabschnitt des Eingriffsabschnitts angeordnet ist, muss der Kontaktabschnitt des elastischen Elements nicht während des gesamten In-Eingriff-Bringens des Befestigungselements mit dem Gegenstück mit diesem in Kontakt sein, sondern das elastische Element ist nur kurz vor Vollendung der Verbindung bzw. des Eingriffs zwischen dem elastischen Element und dem Gegenstück mit dem Gegenstück in Kontakt. Im Wesentlichen kann also das Eingreifen des Befestigungselements mit dem Gegenstück über den Eingriffsabschnitt und das Kontaktieren des elastischen Elements mit dem Gegenstück an verschiedenen axialen Bereichen des Befestigungselements bzw. des Gegenstücks erfolgen.

Vorzugsweise steht der Kontaktabschnitt des nicht-vorgespannten elastischen Elements in der radialen Richtung über den Eingriffsabschnitt des Befestigungselements über, vorzugsweise um mindestens 1 mm und weiter vorzugsweise um mindestens 3 mm. Bei einer derartigen Ausbildung des Kontaktabschnitts kann der gewünschte Kontakt mit dem Gegenstück gewährleistet werden.

Weiter vorzugsweise steht der Endabschnitt über den Eingriffsabschnitt in radialer Richtung und/oder der Kontaktabschnitt des nicht-vorgespannten elastischen Elements in radialer Richtung über den Endabschnitt über. Diese Ausbildung führt die erfindungsgemäßen Vorteile besonders gut aus.

Der Endabschnitt weist vorzugsweise zumindest eine Aussparung auf, an die das elastische Element angrenzt. Weiter vorzugsweise weist der Endabschnitt zumindest zwei benachbarte Aussparungen auf, die durch das elastische Element voneinander getrennt sind. Das elastische Element kann sich in die Aussparung(en) erstrecken, wenn es vorgespannt ist. Mit anderen Worten erstreckt sich die Aussparung zumindest in eine Richtung von dem elastischen Element aus, in die das elastische Element vorspannbar ist.

Wenn zwei Aussparungen vorgesehen sind, die durch das elastische Element voneinander getrennt sind, kann das elastische Element sowohl bei Drehung in die Verbindungsrichtung als auch bei Drehung in die der Verbindungsrichtung entgegengesetzte Richtung in der Aussparung aufgenommen werden.

Das Befestigungselement kann mehrere elastische Elemente aufweisen, die vorzugsweise gleichmäßig zueinander beabstandet sind.

Insbesondere ist das Befestigungselement als Schraube ausgebildet, wobei der Eingriffsabschnitt als Gewinde ausgebildet ist und der Endabschnitt als Schraubenkopf ausgebildet ist.

Der Endabschnitt bzw. der Schraubenkopf kann einen Abschnitt zum Eingriff eines Befestigungswerkzeugs zum Erzeugen der Drehbewegung des Befestigungselements um die Drehachse herum aufweisen.

Das Gegenstück ist vorzugsweise derart ausgebildet, dass es eine Ausnehmung zum zumindest abschnittsweisen Aufnehmen des elastischen Elements, bevorzugt auch des Endabschnitts des Befestigungselements, aufweist. Wenn das Gegenstück eine Ausnehmung aufweist, in der das elastische Element aufgenommen werden kann, hat dies den Vorteil, dass auf zuverlässige Weise der Kontakt zwischen dem Kontaktabschnitt und dem Gegenstück herstellbar ist. Das Gegenstück kann dabei integral ausgebildet sein; es ist aber auch denkbar, dass der Abschnitt mit der Ausnehmung separat zu dem Eingriffsabschnitt am Gegenstück ausgebildet ist.

Die Ausnehmung und der Endabschnitt können jeweils im Wesentlichen kreisförmig ausgebildet sein, der Radius der Ausnehmung kann größer als der des Endabschnitts sein und das nicht-vorgespannte elastische Element kann derart radial über den Endabschnitt vorstehen, dass das vorgespannte elastische Element zumindest abschnittsweise in Kontakt mit der Ausnehmung ist.

Die Tiefe der Ausnehmung kann in Richtung der Drehachse im Wesentlichen der Dicke des Endabschnitts, vorzugsweise auch der Dicke des elastischen Elements, in Richtung der Drehachse entsprechen. Dadurch kann die gewünschte Kontaktierung zwischen dem Festigungselement und dem Gegenstück gewährleistet werden.

Weitere Merkmale und Vorteile der Erfindung werden anhand der nachfolgenden ausführlichen Beschreibung noch näher ersichtlich werden.

### Kurze Beschreibung der Zeichnungen

- Fig. 1: zeigt eine Draufsicht auf einen erfindungsgemäßen Satz aus Befestigungselement mit einem Gegenstück;
- Fig. 2: zeigt eine Perspektivansicht eines Befestigungselements eines erfindungsgemäßen Satzes; und
- Fig. 3: zeigt eine Perspektivansicht eines erfindungsgemäßen Satzes aus Befestigungselement und Gegenstück.

### Ausführliche Beschreibung bevorzugter Ausführungsformen

Eine bevorzugte Ausführungsform der vorliegenden Erfindung wird nachfolgend ausführlich unter Bezugnahme auf die begleitenden Zeichnungen beschrieben.

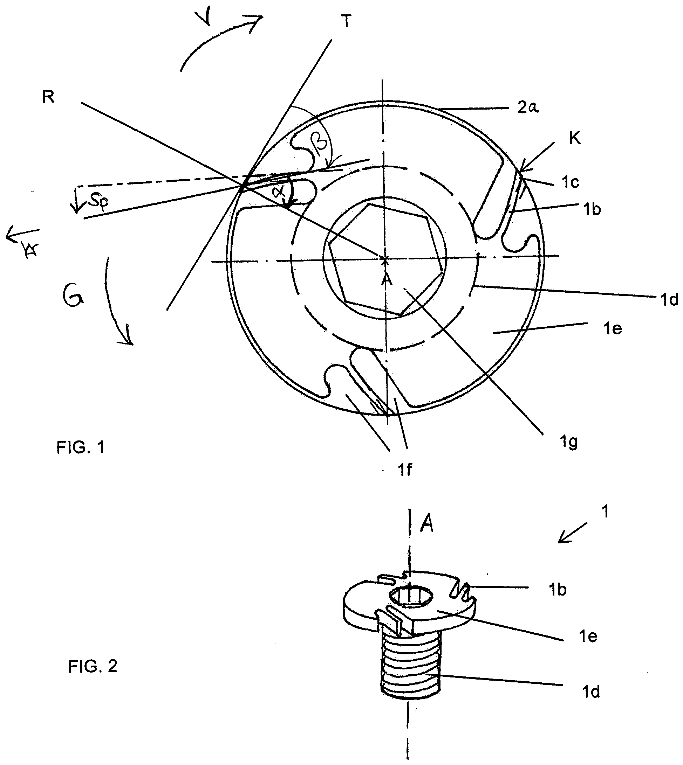

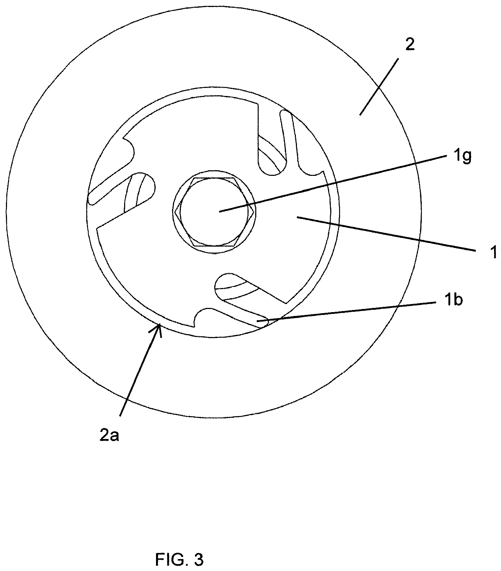

Figur 1 zeigt ein Befestigungselement 1 zum Verhindern von ungewolltem Lösen einer Verbindung zwischen dem Befestigungselement 1 und dem Gegenstück 2. Das Gegenstück 2 weist eine Ausnehmung 2a auf, die kreisförmig ausgebildet ist, was auch aus Figur 3 ersichtlich ist. Das Befestigungselement 1 weist ein elastisches Element 1b auf, das als Federelement ausgebildet sein kann, das wiederum einen Kontaktabschnitt 1c zum Kontaktieren des Gegenstücks 2 aufweist. Dabei ist das elastische Element 1b derart ausgebildet, dass es zumindest abschnittsweise winklig zu einer auf die Drehachse A bezogenen Radialen R in einer Ebene senkrecht zur Drehachse A angeordnet ist.

Figur 1 zeigt drei elastische Elemente 1b, die gleichmäßig zueinander bezüglich des Umfangs des Endabschnitts 1e verteilt angeordnet sind. In Figur 1 ist jedes elastische Element 1b in dem vorgespannten Zustand gezeigt. Der nicht-vorgespannte Zustand ist gestrichelt angedeutet.

Das elastische Element 1b weist in der in den Figuren gezeigten Ausführungsform sowohl im vorgespannten als auch im nicht-vorgespannten Zustand eine derartige Ausbildung auf, dass es winkelig zu einer Radialen R in einer Ebene senkrecht zu der Drehachse A verläuft, sodass im Kontaktpunkt K, in dem der Kontaktabschnitt 1c das Gegenstück 2 kontaktiert, bzw. der Winkel α zwischen dem elastischen Element 1b und der Radialen R in Verbindungsrichtung V 0°<α<90° erfüllt. Der Winkel α verläuft im Uhrzeigersinn von dem elastischen Element 1b zu der Radialen R hin, wie in Figur 1 angedeutet. Dies entspricht der Verbindungsrichtung V, also der Richtung im Uhrzeigersinn.

In Figur 1 ist auch der Winkel β eingezeichnet, in dem das elastische Element 1b im verbundenen Zustand in Verbindungsrichtung V relativ zur auf die Drehachse A bezogenen Tangente T im Kontaktpunkt K in der Ebene senkrecht zur Drehachse A angeordnet ist, und der 0°<β<90° erfüllt. Der Winkel β verläuft in Verbindungsrichtung bzw. im Uhrzeigersinn von der Tangente T zu dem elastischen Element 1b hin, wie in Figur 1 angedeutet. Die Winkel α und β betragen zusammen 90°, wobei der Winkel α vorzugsweise zwischen 5° und 45° liegt und der Winkel β zwischen 45° und 85° liegt.

Auf das elastische Element 1b kann eine Vorspannung ausgeübt werden, indem es in eine Richtung Sp relativ zum nicht-vorgespannten Zustand ausgelenkt wird. Die Richtung Sp unterscheidet sich von der Richtung Kr, in die eine Kraft in das elastische Element 1b bei Drehung des Befestigungselements 1 eingeleitet werden kann. Die Krafteinleitung in das elastische Element 1b bei Drehung des Befestigungselements 1 um die Drehachse A in Verbindungsrichtung V ist kleiner (bzw. vernachlässigbar bzw. Null) als bei Drehung des Befestigungselements in die der Verbindungsrichtung entgegengesetzten Richtung G.

Das elastische Element 1b kommt mit dem Kontaktabschnitt 1c in Kontakt mit dem Gegenstück 2 bzw. der Ausnehmung 2a des Gegenstücks. Dabei ist das elastische Element 1b im vorgespannten Zustand derart relativ zu dem Gegenstück 2 angeordnet, dass der Kontaktabschnitt 1c und das Gegenstück 2 zumindest bei anfänglicher Drehung des Befestigungselements 1 in die der Verbindungsrichtung entgegengesetzten Richtung G kraftschlüssig verbunden sind. Mit anderen Worten ist die im Kontaktpunkt K ausgeübte Haftreibung bei anfänglicher Drehung des Befestigungselements entgegengesetzt zur Verbindungsrichtung V so groß, dass sie durch Einflüsse wie Vibrationen oder Wechsellasten, die auf das Befestigungselement bzw. das Gegenstück wirken, nicht überwunden werden kann.

Das elastische Element 1b ist länglich als Bein ausgebildet und weist eine Länge von mindestens 3 mm und weniger als 20 mm auf. Das elastische Element 1b kann ein Metall aufweisen bzw. vollständig aus Metall bestehen.

Das elastische Element 1b ist als Biegefeder ausgebildet, sodass die Rückstellkraft auf das vorgespannte Element nahezu senkrecht zu der Längsrichtung des elastischen Elements 1b wirkt.

In der in den Figuren gezeigten Ausführungsform ist das elastische Element 1b integral mit dem Befestigungselement 1 ausgebildet. Das elastische Element 1b kann aber auch separat ausgebildet sein und an dem Befestigungselement 1 angeordnet sein.

Figur 2 zeigt das Befestigungselement 1, das als Schraube ausgebildet ist, wobei der Eingriffsabschnitt 1d, mit dem das Befestigungselement mit dem Gegenstück 2 in Eingriff kommt, als Gewinde ausgebildet ist. Der Endabschnitt 1e ist als Schraubenkopf ausgebildet ist, wobei der Eingriffsabschnitt 1d an einem Ende den als Schraubenkopf ausgebildeten Endabschnitt 1e aufweist, an dem das elastische Element 1b angeordnet ist.

Der Endabschnitt 1e kann kreisförmig ausgebildet sein, ebenso wie die in Figur 2 nicht gezeigte Ausnehmung 2a des Gegenstücks 2. Im nicht-vorgespannten Zustand kann der Kontaktabschnitt 1c in der radialen Richtung R, die sich von der Drehachse A in Richtung der Radialen R erstreckt, über den Eingriffsabschnitt 1d des Befestigungselements 1 um mindestens 1 mm bzw. 5 mm überstehen. Dabei kann der Endabschnitt 1e über den Eingriffsabschnitt 1d in radialer Richtung R überstehen und der Kontaktabschnitt 1c des nicht-vorgespannten elastischen Elements 1b in radialer Richtung R über den Endabschnitt 1e vorstehen.

Der Endabschnitt 1e weist in der in den Figuren gezeigten Ausführungsform zwei benachbarte Aussparungen 1f auf, die durch das elastische Element 1b voneinander getrennt sind. Das elastische Element 1b kann sich, wenn es vorgespannt ist, in eine dieser gegenüberliegenden Aussparungen erstrecken.

Des Weiteren weist das Befestigungselement einen Abschnitt 1g zum Eingriff eines Befestigungswerkzeugs zum Erzeugen der Drehbewegung des Befestigungselements um die Drehachse A auf, wie in Figuren 1 und 3 gezeigt.

Die Ausnehmung 2a des Gegenstücks 2 ist so ausgebildet, dass sie das elastische Element 1b und den gesamtem Endabschnitt 1e aufnehmen kann. Dabei ist der Radius der Ausnehmung 2a größer als der Radius des Endabschnitts 1e, und das nicht-vorgespannte elastische Element, das über den Endabschnitt 1e radial übersteht, ist so angeordnet, dass das vorgespannte elastische Element 1b zumindest abschnittsweise in Kontakt mit der Ausnehmung 2a ist.

Die Tiefe der Ausnehmung 2a in Richtung der Drehachse A entspricht im Wesentlichen der Dicke des Endabschnitts 1e bzw. des elastischen Elements 1b in Richtung der Drehachse A. So kann das Befestigungselement 1, wenn es mit dem Gegenstück 2 verbunden ist, vollständig in der Ausnehmung in Richtung der Drehachse A aufgenommen sein, ohne überzustehen.

Das Gegenstück 2 kann integral ausgebildet sein. Es kann aber auch einen Abschnitt mit der Ausnehmung 2a aufweisen, der separat zum Eingriffsabschnitt des Gegenstücks (nicht gezeigt) ausgebildet ist, sodass im Wesentlichen zwei getrennte Abschnitte das Gegenstück 2 ausbilden.

## Patentansprüche

1. Satz aus einem Befestigungselement (1) und einem Gegenstück (2), wobei das Befestigungselement (1) zum Verhindern von ungewolltem Lösen einer Verbindung zwischen dem Befestigungselement (1) und einem Gegenstück (2) dient, wobei das Befestigungselement eingerichtet ist, in Eingriff mit dem Gegenstück (2) über eine Drehbewegung des Befestigungselements um eine Drehachse (A) in eine Verbindungsrichtung (V) zu kommen, und das Befestigungselement aufweist:
ein elastisches Element (1b) mit einem Kontaktabschnitt (1c) zum Kontaktieren des Gegenstücks (2),
wobei das elastische Element derart ausgebildet ist, dass es zumindest abschnittsweise winkelig zu einer auf die Drehachse (A) bezogenen Radialen (R) in einer Ebene senkrecht zur Drehachse angeordnet ist,
wobei das Befestigungselement (1) und das Gegenstück (2) derart ausgebildet sind, dass im verbundenen Zustand des Befestigungselements (1) und des Gegenstücks der Kontaktabschnitt (1c) das Gegenstück (2) in einem Kontaktpunkt (K) kontaktiert und dass eine auf die Drehachse (A) bezogene Tangente (T) im Kontaktpunkt (K) zumindest zu einem Abschnitt des elastischen Elements in der Ebene senkrecht zu der Drehachse in einem Winkel ungleich 90° verläuft, wodurch die radialen Kräfte beim Drehen des Befestigungselements in die Verbindungsrichtung und in eine zu der Verbindungsrichtung entgegengesetzte Richtung unterschiedlich sind, sodass eine Krafteinleitung in das elastische Element bei Drehung des Befestigungselements um die Drehachse in Verbindungsrichtung (V) kleiner als bei Drehung des Befestigungselements in die der Verbindungsrichtung entgegengesetzte Richtung (G) ist, und
wobei das elastische Element (1b) derart ausgebildet ist, dass der Kontaktabschnitt (1c) des vorgespannten elastischen Elements und das Gegenstück (2) zumindest bei anfänglicher Drehung des Befestigungselements in die der Verbindungsrichtung entgegengesetzte Richtung (G) kraftschlüssig verbunden sind.

2. Satz nach Anspruch 1, bei dem das Gegenstück (2) eine Ausnehmung (2a) zum zumindest abschnittsweisen Aufnehmen des elastischen Elements (1b), bevorzugt auch des Endabschnitts (1e), aufweist.

3. Satz nach Anspruch 2, bei dem die Ausnehmung (2a) und der Endabschnitt (1e) jeweils im Wesentlichen kreisförmig ausgebildet sind, der Radius der Ausnehmung (2a) größer als der des Endabschnitts (1e) ist und das nicht-vorgespannte elastische Element (1b) derart radial über den Endabschnitt (1e) übersteht, dass das vorgespannte elastische Element zumindest abschnittsweise in Kontakt mit der Ausnehmung (2a) ist.

4. Satz nach einem der vorhergehenden Ansprüche, bei dem das elastische Element (1b) derart ausgebildet ist, dass Vorspannung auf das elastische Element derart aufbringbar ist, dass Kontakt zwischen dem Kontaktabschnitt (1c) und dem Gegenstück (2) in dem Kontaktpunkt (K) sichergestellt werden kann.

5. Satz nach einem der vorhergehenden Ansprüche, bei dem das elastische Element, vorzugsweise auch in einem nicht-vorgespannten Zustand, derart winkelig zur Radialen verläuft, dass zumindest im Kontaktpunkt (K) der Winkel α zwischen dem elastischen Element (1b) und der Radialen (R) in Verbindungsrichtung (V) 0° < α < 90°, vorzugsweise 5° < α < 85°, weiter vorzugsweise 5° < α < 45°, erfüllt.

6. Satz nach einem der vorhergehenden Ansprüche, bei dem das elastische Element (1b) derart ausgebildet ist, dass eine Richtung (Sp), in die das elastische Element vorspannbar ist, unterschiedlich zu einer Richtung (Kr) ist, in die die Krafteinleitung in das elastische Element bei Drehung des Befestigungselements erfolgen kann.

7. Satz nach einem der vorhergehenden Ansprüche, bei dem das elastische Element als Federelement und im Wesentlichen länglich, insbesondere als Bein, weiter insbesondere als Biegefeder, ausgebildet ist.

8. Satz nach einem der vorhergehenden Ansprüche, bei dem das Befestigungselement einen Eingriffsabschnitt (1d) zum Eingriff mit dem Gegenstück (2) aufweist, wobei der Eingriffsabschnitt (1d) an einem Ende einen vorzugsweise zumindest abschnittsweise kreisförmigen Endabschnitt (1e) aufweist, an dem das elastische Element (1b) angeordnet ist.

9. Satz nach Anspruch 8, bei dem der Kontaktabschnitt (1c) des nicht-vorgespannten elastischen Elements in der radialen Richtung (R) über den Eingriffsabschnitt (1d) des Befestigungselements (1) übersteht, vorzugsweise um mindestens 1 mm, weiter vorzugsweise mindestens 3 mm.

10. Satz nach Anspruch 8 oder 9, bei dem der Endabschnitt (1e) über den Eingriffsabschnitt (1d) in radialer Richtung (R) übersteht und/oder der Kontaktabschnitt (1c) des nicht-vorgespannten elastischen Elements in radialer Richtung (R) über den Endabschnitt (1e) übersteht.

11. Satz nach einem der vorhergehenden Ansprüche 8 bis 10, bei dem der Endabschnitt (1e) zumindest eine Aussparung (1f) aufweist, an die das elastische Element (1b) angrenzt, vorzugsweise zwei benachbarte Aussparungen (1f) aufweist, die durch das elastische Element (1b) voneinander getrennt sind, und in die sich das vorgespannte elastische Element erstrecken kann.

12. Satz nach einem der vorhergehenden Ansprüche, wobei das Befestigungselement mehrere elastische Elemente (1b) aufweist, die vorzugsweise gleichmäßig zueinander beabstandet sind.

13. Satz nach einem der Ansprüche 8 bis 11, bei dem das Befestigungselement das als Schraube ausgebildet ist, wobei
der Eingriffsabschnitt (1d) als Gewinde ausgebildet ist und
der Endabschnitt (1e) als Schraubenkopf ausgebildet ist.

14. Verwendung eines Befestigungselements (1) zum Verhindern von ungewolltem Lösen einer Verbindung zwischen dem Befestigungselement (1) und einem Gegenstück (2), wobei das Befestigungselement eingerichtet ist, in Eingriff mit dem Gegenstück (2) über eine Drehbewegung des Befestigungselements um eine Drehachse (A) in eine Verbindungsrichtung (V) zu kommen,
wobei das Befestigungselement ein elastisches Elementmit einem Kontaktabschnitt (1c) zum Kontaktieren des Gegenstücks (2) in einem Kontaktpunkt (K) aufweist, und der Kontaktabschnitt (1c) das Gegenstück (2) im verbundenen Zustand des Befestigungselements und des Gegenstücks kontaktiert,
das elastische Element im verbundenen Zustand derart angeordnet ist, dass eine auf die Drehachse (A) bezogene Tangente (T) im Kontaktpunkt (K) zumindest zu einem Abschnitt des elastischen Elements in einer Ebene senkrecht zu der Drehachse in einem Winkel ungleich 90° verläuft, wodurch die radialen Kräfte beim Drehen des Befestigungselements in die Verbindungsrichtung und in eine zu der Verbindungsrichtung entgegengesetzte Richtung unterschiedlich sind, sodass eine Krafteinleitung in das elastische Element bei Drehung des Befestigungselements um die Drehachse in Verbindungsrichtung (V) kleiner als bei Drehung des Befestigungselements in die der Verbindungsrichtung entgegengesetzte Richtung (G) ist, und
das elastische Element (1b) derart ausgebildet ist, dass der Kontaktabschnitt (1c) des vorgespannten elastischen Elements und das Gegenstück (2) zumindest bei anfänglicher Drehung des Befestigungselements in die der Verbindungsrichtung entgegengesetzte Richtung (G) kraftschlüssig verbunden sind.

15. Verwendung des Satzes nach einem der Ansprüche 1 bis 13 zur Verbindung von Implantatkomponenten, insbesondere einer vorzugsweise winkelstabilen Knochenplatte.

## Claims

1. A set consisting of a fastening element (1) and a counterpart (2), wherein the fastening element (1) serves to prevent the unwanted release of a connection between the fastening element (1) and a counterpart (2), wherein the fastening element is set up to come into engagement with the counterpart (2) via a rotational movement of the fastening element about an axis of rotation (A) in a connection direction (V), and the fastening element has:
an elastic element (1b) with a contact portion (1c) for contacting the counterpart (2),
wherein the elastic element is configured such that it is arranged at least in portions at an angle to a radial (R) relative to the axis of rotation (A) on a plane perpendicular to the axis of rotation,
wherein the fastening element (1) and the counterpart (2) are configured such that in the connected state of the fastening element (1) and of the counterpart the contact portion (1c) contacts the counterpart (2) in a contact point (K) and that a tangent (T) in the contact point (K) relative to the axis of rotation (A) extends at least to a portion of the elastic element on the plane perpendicular to the axis of rotation at an angle unequal to 90°, as a result of which the radial forces during rotation of the fastening element in the connection direction and in an opposite direction to the connection direction are different, so that a transmission of force to the elastic element during rotation of the fastening element about the axis of rotation in the connection direction (V) is smaller than during rotation of the fastening element in an opposite direction (G) to the connection direction, and
wherein the elastic element (1b) is configured such that the contact portion (1c) of the pre-stressed elastic element and the counterpart (2) at least during the initial rotation of the fastening element in the opposite direction (G) to the connection direction are non-positively connected.

2. The set according to claim 1, in which the counterpart (2) has a recess (2a) for accommodating the elastic element (1b) at least in portions, preferably also the end portion (1e).

3. The set according to claim 2, in which the recess (2a) and the end portion (1e) are each configured substantially circular, the radius of the recess (2a) is greater than that of the end portion (1e) and the non-pre-stressed elastic element (1b) protrudes radially above the end portion (1e) such that the pre-stressed elastic element is in contact with the recess (2a) at least in portions.

4. The set according to any of the foregoing claims, in which the elastic element (1b) is configured such that the prestress can be applied to the elastic element such that contact between the contact portion (1c) and the counterpart (2) in the contact point (K) can be ensured.

5. The set according to any of the foregoing claims, in which the elastic element, preferably also in a non-pre-stressed state, extends at an angle to the radial such that at least in the contact point (K) the angle α between the elastic element (1b) and the radial (R) in the connection direction (V) satisfies 0° < α < 90°, preferably 5° < α < 85°, further preferably 5° < α < 45°.

6. The set according to any of the foregoing claims, in which the elastic element (1b) is configured such that a direction (Sp), in which the elastic element can be pre-stressed, is different to a direction (Kr), in which the transmission of force to the elastic element can occur during rotation of the fastening element.

7. The set according to any of the foregoing claims, in which the elastic element is configured as a spring element and substantially elongated, in particular as a leg, further in particular as a torsion spring.

8. The set according to any of the foregoing claims, in which the fastening element has an engagement portion (1d) for engagement with the counterpart (2), wherein the engagement portion (1d) has on one end a circular end portion (1e) preferably at least in portions, on which the elastic element (1b) is arranged.

9. The set according to claim 8, in which the contact portion (1c) of the non-pre-stressed elastic element protrudes above the engagement portion (1d) of the fastening element (1) in the radial direction (R), preferably by at least 1 mm, further preferably by at least 3 mm.

10. The set according to claim 8 or 9, in which the end portion (1e) protrudes above the engagement portion (1d) in the radial direction (R) and/or the contact section (1c) of the non-pre-stressed elastic element protrudes above the end portion (1e) in the radial direction (R).

11. The set according to any of the foregoing claims 8 to 10, in which the end portion (1e) has at least one notch (If), at which the elastic element (1b) adjoins, preferably two neighboring notches (If), which are separated from each other by the elastic element (1b) and which can extend to the pre-stressed elastic element.

12. The set according to any of the foregoing claims, wherein the fastening element has a plurality of elastic elements (1b) which are preferably uniformly spaced apart from each other.

13. The set according to any claims 8 to 11, in which the fastening element is configured as a screw, wherein
the engagement element (1d) is configured as a thread and
the end portion (1e) is configured as a screw head.

14. The use of a fastening element (1) to prevent unwanted release of a connection between the fastening element (1) and a counterpart (2), wherein the fastening element is set up to come into engagement with the counterpart (2) via a rotational movement of the fastening element about an axis of rotation (A) in a connection direction (V),
wherein the fastening element has an elastic element with a contact portion (1c) for contacting the counterpart (2) in a contact point (K), and the contact portion (1c) contacts the counterpart (2) in the connected state of the fastening element and of the counterpart,
the elastic element in the connected state is arranged such that and that a tangent (T) in the contact point (K) relative to the axis of rotation (A) extends at least to a portion of the elastic element on the plane perpendicular to the axis of rotation at an angle unequal to 90°, as a result of which the radial forces during rotation of the fastening element in the connection direction and in an opposite direction to the connection direction are different, so that a transmission of force to the elastic element during rotation of the fastening element about the axis of rotation in the connection direction (V) is smaller than during rotation of the fastening element in an opposite direction (G) to the connection direction, and
the elastic element (1b) is configured such that the contact portion (1c) of the pre-stressed elastic element and the counterpart (2) at least during the initial rotation of the fastening element in the opposite direction (G) to the connection direction are non-positively connected.

15. The use of the set according to any of claims 1 to 13 for connecting implant components, in particular of a preferably fixed-angle bone plate.

## Revendications

1. Jeu composé d'un élément de fixation (1) et d'une pièce complémentaire (2), dans lequel l'élément de fixation (1) sert à empêcher une libération involontaire d'une liaison entre l'élément de fixation (1) et une pièce complémentaire (2), dans lequel l'élément de fixation est conçu pour venir en prise avec la pièce complémentaire (2) par l'intermédiaire d'un mouvement rotatif de l'élément de fixation autour d'un axe de rotation (A) dans une direction de liaison (V), et l'élément de fixation présente :
un élément élastique (1b) avec une partie de contact (1c) pour la mise en contact de la pièce complémentaire (2),
dans lequel l'élément élastique est réalisé de telle sorte qu'il est disposé au moins sur certaines parties de manière angulaire par rapport à une radiale (R) par rapport à l'axe de rotation (A) dans un plan perpendiculairement à l'axe de rotation,
dans lequel l'élément de fixation (1) et la pièce complémentaire (2) sont réalisés de telle sorte que, dans l'état relié de l'élément de fixation (1) et de la pièce complémentaire, la partie de contact (1c) est en contact avec la pièce complémentaire (2) dans un point de contact (K) et qu'une tangente (T) par rapport à l'axe de rotation (A) dans le point de contact (K) s'étend au moins par rapport à une partie de l'élément élastique dans le plan perpendiculairement à l'axe de rotation selon un angle différent de 90°, ce qui a pour effet que les forces radiales, lors de la rotation de l'élément de fixation, sont différentes dans la direction de liaison et dans une direction opposée à la direction de liaison, de sorte qu'une introduction de force dans l'élément élastique lors de la rotation de l'élément de fixation autour de l'axe de rotation dans la direction de liaison (V) est plus petite que lors de la rotation de l'élément de fixation dans la direction (G) opposée à la direction de liaison, et
dans lequel l'élément élastique (1b) est réalisé de telle sorte que la partie de contact (1c) de l'élément élastique précontraint et la pièce complémentaire (2) sont reliées à force au moins au début de la rotation de l'élément de fixation dans la direction (G) opposée à la direction de liaison.

2. Jeu selon la revendication 1, pour lequel la pièce complémentaire (2) présente un creux (2a) pour la réception au moins sur certaines parties de l'élément élastique (1b), de préférence également de la partie d'extrémité (le).

3. Jeu selon la revendication 2, pour lequel le creux (2a) et la partie d'extrémité (1e) sont réalisés respectivement sensiblement de manière circulaire, le rayon du creux (2a) est plus grand que celui de la partie d'extrémité (1e) et l'élément élastique (1b) non précontraint fait saillie radialement de la partie d'extrémité (le), de telle sorte que l'élément élastique précontraint est en contact avec le creux (2a) au moins sur certaines parties.

4. Jeu selon l'une quelconque des revendications précédentes, pour lequel l'élément élastique (1b) est réalisé de telle sorte qu'une précontrainte peut être appliquée sur l'élément élastique, de telle sorte qu'un contact entre la partie de contact (1c) et la pièce complémentaire (2) dans le point de contact (K) peut être garanti.

5. Jeu selon l'une quelconque des revendications précédentes, pour lequel l'élément élastique, de préférence également dans un état non précontraint, s'étend de manière angulaire par rapport à la radiale, de telle sorte qu'au moins dans le point de contact (K), l'angle α entre l'élément élastique (1b) et la radiale (R) dans la direction de liaison (V) satisfait 0° < α < 90°, de préférence 5° < α < 85°, plus préférablement 5° < α < 45°.

6. Jeu selon l'une quelconque des revendications précédentes, pour lequel l'élément élastique (1b) est réalisé de telle sorte qu'une direction (Sp), dans laquelle l'élément élastique peut être précontraint, est différente d'une direction (Kr), dans laquelle l'introduction de force dans l'élément élastique peut s'effectuer lors de la rotation de l'élément de fixation.

7. Jeu selon l'une quelconque des revendications précédentes, pour lequel l'élément élastique est réalisé sous la forme d'un élément ressort et sensiblement de manière allongée, en particulier sous la forme d'une jambe, plus particulièrement sous la forme d'un ressort de flexion.

8. Jeu selon l'une quelconque des revendications précédentes, pour lequel l'élément de fixation présente une partie de mise en prise (1d) pour la mise en prise avec la pièce complémentaire (2), dans lequel la partie de mise en prise (1d) présente à une extrémité une partie d'extrémité (1e) de préférence circulaire au moins sur certaines parties, sur laquelle l'élément élastique (1b) est disposé.

9. Jeu selon la revendication 8, pour lequel la partie de contact (1c) de l'élément élastique non précontraint fait saillie de la partie de mise en prise (1d) de l'élément de fixation (1), de préférence d'au moins 1 mm, plus préférablement d'au moins 3 mm dans la direction radiale (R).

10. Jeu selon la revendication 8 ou 9, pour lequel la partie d'extrémité (1e) fait saillie de la partie de mise en prise (1d) dans la direction radiale (R) et/ou la partie de contact (1c) de l'élément élastique non précontraint fait saillie de la partie d'extrémité (1e) dans la direction radiale (R).

11. Jeu selon l'une quelconque des revendications précédentes 8 à 10, pour lequel la partie d'extrémité (1e) présente au moins un évidement (1f), auquel l'élément élastique (1b) est adjacent, de préférence deux évidements (1f) voisins, qui sont séparés l'un de l'autre par l'élément élastique (1b), et dans lequel l'élément élastique précontraint peut s'étendre.

12. Jeu selon l'une quelconque des revendications précédentes, dans lequel l'élément de fixation présente plusieurs éléments élastiques (1b), qui sont espacés les uns des autres de préférence de manière régulière.

13. Jeu selon l'une quelconque des revendications 8 à 11, pour lequel l'élément de fixation est réalisé sous la forme d'une vis, dans lequel
la partie de mise en prise (1d) est réalisée sous la forme d'un filet et
la partie d'extrémité (1e) est réalisée sous la forme d'une tête de vis.

14. Utilisation d'un élément de fixation (1) pour empêcher une libération involontaire d'une liaison entre l'élément de fixation (1) et une pièce complémentaire (2), dans laquelle l'élément de fixation est conçu pour venir en prise avec la pièce complémentaire (2) par l'intermédiaire d'un mouvement rotatif de l'élément de fixation autour d'un axe de rotation (A) dans une direction de liaison (V),
dans laquelle l'élément de fixation présente un élément élastique avec une partie de contact (1c) pour la mise en contact de la pièce complémentaire (2) dans un point de contact (K), et la partie de contact (1c) vient en contact avec la pièce complémentaire (2) dans l'état relié de l'élément de fixation et de la pièce complémentaire,
l'élément élastique dans l'état relié est disposé de telle sorte qu'une tangente (T) par rapport à l'axe de rotation (A) dans le point de contact (K) s'étend au moins par rapport à une partie de l'élément élastique dans un plan perpendiculairement à l'axe de rotation selon un angle différent de 90°, ce qui a pour effet que les forces radiales, lors de la rotation de l'élément de fixation, sont différentes dans la direction de liaison et dans une direction opposée à la direction de liaison, de sorte qu'une introduction de force dans l'élément élastique lors de la rotation de l'élément de fixation autour de l'axe de rotation dans la direction de liaison (V) est plus petite que lors de la rotation de l'élément de fixation dans la direction (G) opposée à la direction de liaison, et
l'élément élastique (1b) est réalisé de telle sorte que la partie de contact (1c) de l'élément élastique précontraint et la pièce complémentaire (2) sont reliées à force au moins au début de la rotation de l'élément de fixation dans la direction (G) opposée à la direction de liaison.

15. Utilisation du jeu selon l'une quelconque des revendications 1 à 13 pour la liaison de composants d'implant, en particulier d'une plaque osseuse de préférence angulairement stable.
